# FASCICULE DE BREVET EUROPEEN

(11) **EP 1 505 953 B1**
(45) Date de publication et mention de la délivrance du brevet: **23.07.2014**
(21) Numéro de dépôt: 03740688.1
(22) Date de dépôt: 30.04.2003
(51) Int. Cl.: A61K 8/60, A61Q 19/08, A61Q 19/00

(54) **NOUVELLES COMPOSITIONS A USAGE CUTANE A BASE DE POLYOLS-GLYCOSIDES**
HAUTPFLEGEMITTEL AUF BASIS VON POLYOLGLYKOSIDEN
NOVEL POLYOL-GLYCOSIDE COMPOSITIONS FOR THE SKIN

(30) Priorité: 07.05.2002 FR 0205681
(43) Date de publication de la demande: 16.02.2005
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: STOLTZ, Corinne, F-94320 Thiais (FR); BOITEUX, Jean-Pierre, F-81710 Saix (FR); MILIUS, Alain, F-06000 Nice (FR); ROLLAND, Hervé, F-81100 Castres (FR); TABACCHI, Guy, F-75007 Paris (FR); GARCIA, Christine, F-81100 Castres (FR)
(74) Mandataire: Conan, Philippe Claude
(86) Numéro de dépôt international: PCT/FR2003/001364
(87) Numéro de publication internationale: WO 2003/094864

(56) Documents cités:
- EP-A- 0 770 378
- FR-A- 2 730 409
- DATABASE CA [en ligne] CHEMICAL ABSTRACTS SERVICE, COLUMBUS, OHIO, US; MATSUZAWA, SACHIYO ET AL: "Moisturizing cosmetics containing saccharides" retrieved from STN Database accession no. 127:23570 XP002229201 & JP 09 077650 A (AJINOMOTO CO., INC., JAPAN) 25 mars 1997 (1997-03-25)
- DATABASE CA [en ligne] CHEMICAL ABSTRACTS SERVICE, COLUMBUS, OHIO, US; UEHARA, SHIZUKA ET AL: "Rough skin -preventing and antiaging cosmetics" retrieved from STN Database accession no. 132:283941 XP002229202 & JP 2000 119155 A (KOSEI CO., LTD., JAPAN) 25 avril 2000 (2000-04-25)
- PATENT ABSTRACTS OF JAPAN vol. 012, no. 283 (C-518), 3 août 1988 (1988-08-03) & JP 63 063390 A (KAO CORP), 19 mars 1988 (1988-03-19)
- DATABASE WPI Section Ch, Week 197744 Derwent Publications Ltd., London, GB; Class A96, AN 1977-78724Y XP002229203 & JP 52 114027 A (IIZUKA T), 24 septembre 1977 (1977-09-24)

## Description

La présente invention a pour objet de nouvelles compositions à usage topique à base de xylityl glucoside.

L'invention trouve application préférentiellement dans le domaine cosmétique, mais également dans le domaine dermopharmaceutique ou pharmaceutique, dans le domaine de l'industrie textile par exemple pour le traitement de fibres textiles synthétiques ou naturelles tissées ou tricotées, ou encore dans le domaine de l'industrie papetière par exemple pour la fabrication de papier à usage sanitaire ou domestique.

L'expression "à usage topique" utilisée dans le cadre de la présente description s'entend donc dans son acception la plus large, pour désigner toutes applications directes (cas d'un produit cosmétique, dermopharmaceutique ou pharmaceutique) ou indirectes (cas de fibres textiles ou de papiers) d'une composition sur la peau, ou les muqueuses.

Dans ses applications directes sur la peau , l'invention vise plus spécifiquement des compositions permettant d'améliorer l'intégrité de la peau en procurant un confort cutané.

Par l'expression composition ou substance susceptible d'améliorer "l'intégrité de la peau" on désigne toute composition ou substance présentant des propriétés hydratantes résultant notamment d'une aptitude à renforcer le taux hydrique épidermique en favorisant notamment la synthèse des glycosaminoglycanes et/ou des propriétés restructurantes, résultant notamment d'une aptitude à augmenter la cohésion cellulaire de la peau par stimulation de la synthèse des céramides épidermiques.

On sait que les compositions cosmétiques contiennent généralement des substances hydratantes, comme en particulier des polyols, des polyols éthoxylés ou des protéines hydrolysées.

Parmi les polyols, c'est le glycérol (polyol comprenant trois groupes hydroxyles) qui présente le pouvoir hydratant le plus élevé. Cependant, il a été constaté qu'à dose élevée, celui-ci peut provoquer certaines irritations de la peau et des muqueuses chez des personnes particulièrement sensibles.

La recherche de nouvelles substances hydratantes, mieux tolérées que le glycérol, a notamment conduit à l'utilisation de certains de ses dérivés comme en particulier ses acétals résultant de la condensation avec un sucre réducteur.

Ces dérivés qui présentent effectivement une meilleure tolérance cutanée que le glycérol se caractérisent cependant par un pouvoir hydratant généralement inférieur à celui-ci.

Parmi les acétals de glycérol, les produits d'acétalisation du glycérol et du glucose décrits dans le document EP 0 770 378 semblaient jusqu'à ce jour présenter le meilleur compromis entre pouvoir hydratant et tolérance cutanée.

De plus, JP 63 06 3390 A décrit un procédé de préparation par voie enzymatique du mannosyl erythritol, son activité de rétention de l'hydratation cutanée.

Il a été découvert de façon inattendue, et ceci constitue le fondement de la présente invention, que les glycosides obtenus par acétalisation de certains polyols contenant 5, fonctions hydroxyles, présentent de meilleures propriétés hydratantes que les produits décrits dans le document EP 0770378, tout en ayant une tolérance cutanée identique.

Cette découverte est d'autant plus surprenante, qu'elle va à l'encontre d'un préjugé, puisque l'homme du métier sait que le pouvoir hydratant des polyols diminue lorsque le nombre de fonctions hydroxyles augmente.

En outre, il a été observé, d'une manière tout à fait surprenante, que les glycosides précités présentent des propriétés restructurantes remarquables, se traduisant notamment par une meilleure aptitude à augmenter la cohésion cellulaire de la peau que le glycérol.

Ainsi, selon un premier aspect, la présente invention a pour objet de nouvelles compositions à usage topique, caractérisées en ce qu'elles contiennent une quantité efficace d'un polyol-glycoside obtenu par acétalisation du xylitol de formule : 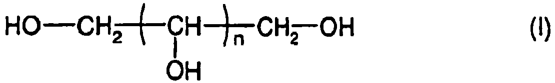 dans laquelle n est un nombre entier égal à 3 avec du glucose

L'expression "quantité efficace" utilisée dans le cadre de la présente demande signifie une quantité suffisante pour fournir à la composition une activité hydratante et/ou restructurante de l'épiderme.

Le xylityl glucoside constitue le composé mis en oeuvre dans le cadre de l'invention.

Les compositions à usage topique selon l'invention peuvent être utilisées dans de nombreux domaines.

Selon une caractéristique particulière, ces compositions seront choisies parmi une composition cosmétique, une composition dermopharmaceutique, une composition pharmaceutique, une composition d'imprégnation pour lingettes.

Selon un deuxième aspect, la présente invention a pour objet l'utilisation telle que définie à la revendication 4 ou 5.

Le polyols-glycoside, dont l'utilisation est préconisée selon la présente invention, pour la réalisation de compositions à usage topique, peuvent être obtenus par diverses voies de synthèse.

Une première vole, dite "synthèse one-pot", consiste à introduire un sucre réducteur et un polyol de formule (I) ou (II) dans un réacteur, selon un rapport stoechiométrique maîtrisé, et à soumettre ce mélange à une réaction d'acétalisation dans des conditions de température et de vide partiel prédéterminées en présence d'un système catalytique acide.

Les composants de ce système catalytique acide seront généralement choisis parmi les acides sulfurique, chlorhydrique, phosphorique, nitrique, hypophosphoreux, méthane-sulfonique, para-toluène sulfonique, trifluoro-méthane sulfonique et les résines échangeuses d'ions acides.

Habituellement, la réaction d'acétalisation sera réalisée à une température de 70 à 130°C, sous un vide de 300 à 20 mbars.

Une deuxième voie de synthèse consiste à :
a) soumettre le polyol de formule (I) à une déshydratation, en présence d'un système catalytique acide, à une température comprise entre 70°C et 130°C, sous vide partiel, avec élimination concomitante de l'eau formée lors du réarrangement intra-moléculaire subi par le polyol ; puis
b) acétaliser le polyol déshydraté ainsi obtenu par dispersion du glucose dans le milieu réactionnel et par maintien de celui-ci à une température comprise entre 80°C et 130°C, sous vide partiel.

Le système catalytique acide utilisé dans cette deuxième voie de synthèse peut être identique à celui évoqué pour la première voie.

Une troisième voie de synthèse par trans-acétalisation consiste à :
a) préparer du butylglucoside par réaction entre le butanol et le glucose en présence d'un système catalytique acide, à une température comprise entre 90°C et 105°C, sous vide partiel, avec élimination concomitante de l'eau formée lors de la réaction ; et
b) ajouter un polyol de formule (I) au milieu réactionnel ainsi obtenu, avec évacuation par distillation sous vide du butanol résiduel, du butanol formé au cours de la réaction de trans-acétalisation, et de l'eau éventuellement générée lors du réarrangement intra-moléculaire dudit polyol.

Le polyols-glycoside utile dans le cadre de la présente invention est un produits stable et hydrosoluble.

De ce fait, il peut être incorporé dans tout type de formulation destinée à un usage topique, ou bien encore dans tout type de support destiné à être mis en contact avec la peau (papier, lingette, textile, dispositif transdermique, etc.).

En particulier, ce produit peut être formulé sous forme de solution, d'émulsion ou de micro-émulsion du type eau-dans-huile (E/H) ou huile-dans-eau (H/E), d'émulsion multiple de type eau-dans-huile-dans-eau (E/H/E) ou huile-dans-eau-dans-huile (H/E/H), de gel, d'hydrodispersion, de bâton solide, de pommade, d'aérosol ou encore sous forme anhydre comme une poudre.

Ce produit peut également être encapsulé, par exemple dans des trames de collagène ou d'autres substances d'encapsulation usuelles, comme par exemple sous forme d'encapsulations de cellulose, dans de la gélatine, dans des matrices de cire ou dans des liposomes.

Le polyols-glycoside utile dans le cadre de la présente invention présente des propriétés hydratantes remarquables et permettent en particulier de renforcer le taux hydrique épidermique et de favoriser la synthèse des glycosaminoglycanes.

Le polyols-glycoside utile dans le cadre de la présente invention présente également des propriétés restructurantes remarquables et permettent en particulier d'augmenter la cohésion cellulaire de la peau. Les lipides épidermiques représentent 10 à 12 % du poids de l'épiderme sec. Ils interviennent dans la perméabilité du *stratum corneum,* dans le phénomène de désquamation et dans la régulation des flux hydriques cutanés. Les céramides sont les composants lipidiques essentiels du *stratum corneum,* notamment le céramide 1, le céramide 3, le céramide 2, le céramide 4, le céramide 5, le céramide 6. Plus précisément, des modifications de la quantité et de la distribution des céramides sont observées dans un grand nombre de pathologies cutanées, en particulier celles associées à des désordres de la kératinisation et de l'hydratation cutanée : psoriasis, dermatose atypique, ichthiose, syndrome Sjogren-Larsson, xérose, eczéma.

Les nouvelles compositions contenant le polyols-glycoside utile dans le cadre de la présente invention permettent d'augmenter de façon significative la néosynthèse des céramides épidermiques, plus précisément du céramide 1 et du céramide 2. Cet accroissement présente un caractère surprenant dans le sens qu'il n'est pas observé dans des conditions expérimentales identiques avec le glycérol.

Par conséquent, ce produit peut être utilisé dans tout type d'application où une action hydratante et/ou restructurante de l'épiderme est recherchée, par exemple pour les soins du visage ou du corps. Il peut également être utilisé dans des systèmes aqueux ou des compositions de tensioactifs destinées au nettoyage de la peau et au lavage des cheveux.

Le polyols-glycoside utile dans le cadre de la présente invention sera généralement utilisé seuls ou en association avec d'autres principes actifs à une dose d'environ 0,01 % à 30 % en poids, de préférence de 0,1 à 10 % en poids dans des formulations cosmétiques ou dermo-pharmaceutiques à activité hydratante et/ou restructurante.

Ces formulations peuvent être des formules à vocation anti age, restructurante, stimulante, anti-radicalaire, anti-oxydante, antipelliculaire, anti-acnéique, apaisante, anti-neuromédiateurs, anti-Substance P, anti-allergique, antidouleur, anti-stress, anti-rides, pro-fermeté, pro-élasticité, cicatrisante, raffermissante, tenseur, amincissante, veinotonique, drainante, anti-rougeur, immunomodulatrice, éclaircissante, revitalisante, ou encore des formules destinées à améliorer le teint de la peau, à stimuler les cellules ou à favoriser la synthèse des protéines cutanées comme le collagène ou la kératine.

Les formulations à activité hydratante et/ou restructurante de l'épiderme incorporant un polyol-glycoside selon l'invention pourront être préparées par les méthodes classiquement utilisées par l'homme du métier dans le domaine de la cosmétologie, ou de la dermo-pharmacie.

Le polyols-glycoside selon l'invention est particulièrement utile pour les peaux fatiguées car ils apportent les éléments nécessaires au dynamisme cellulaire et au maintien des fonctions cutanées. En outre il stimule la régénération cellulaire, permettant à la peau de retrouver éclat et fraîcheur.

Ce polyols-glycoside peut également être utilisé dans les formules destinées à améliorer les échanges cellulaires, l'état de la jonction derme - épiderme ou encore dans les produits solaires, les produits de maquillage, tels que les rouges à lèvre, les fards, les poudres ou dans les produits de traitement ou de coloration des cheveux.

Ce polyols-glycoside peut être associé à tous les types d'adjuvants habituellement utilisés dans les formulations à usage topique, en particulier cosmétiques, ou dermo-pharmaceutiques comme par exemple les corps gras, les solvants organiques, les épaississants et gélifiants, les adoucissants, les antioxydants, les opacifiants, les stabilisants, les moussants, les parfums, les émulsionnants ioniques ou non ioniques, les charges minérales, les agents séquestrants, les agents chelatants, les conservateurs, les filtres chimiques ou minéraux, les huiles essentielles, les matières colorantes, les pigments, les actifs hydrophiles ou lipophiles, les vésicules lipidiques, etc.

Parmi les huiles susceptibles d'être associées à ce polyols-glycoside, on peut citer les paraffines, les isoparaffines, les huiles blanches minérales, les huiles végétales, les huiles animales, les huiles de synthèse, les huiles siliconées et les huiles fluorées.

Parmi les autres matières grasses que l'on peut associer à ce produit, on citera les alcools gras ou les acides gras, les cires et les beurres.

Parmi les agents émulsionnants que l'on peut associer à ce produit, on citera les compositions à base d'alkylpolyglycosides et d'alcools gras décrites dans les brevets US5958431, US6353034, US5888482, US6268400, US5670471.

Parmi les agents gélifiants ou épaississants que l'on peut associer à ce produit, on citera les polymères d'origine naturelle comme les gommes de xanthane, les polysaccharides, les polymères d'origine synthétique tels que les polymères carboxyvinyliques (Carbomer ^{™}), les copolymères acryliques, les polyacrylamides ou d'autres polymères présentés en émulsion inverse et décrits dans les brevets US6197287, US6346239, EP1056805, EP1166771, EP1152023, EP1152022, les dérivés de sucres polyoxyéthylènés (méthylglucose éthoxylé), les silicates mixtes d'aluminium-magnésium et de sodium-magnésium.

Parmi les agents moussants que l'on peut associer à ce produit, on citera les bétaïnes, les sulfobétaïnes, les alkylpolyglucosides, les lipoaminoacides, les lipopeptides, le lauryl éthersulfate de sodium, les alkyl sulfates, les alkyl éthers sulfates, les alkyl éthers carboxylates, les dérivés de lipoprotéines, les dérivés de protéines, les immidazolines, les sulfosuccinates.

Parmi les principes actifs que l'on peut associer aux polyols-glycoside hydratant de l'invention pour potentialiser leurs propriétés, on citera par exemple tout actif présentant déjà des propriétés hydratantes, ou bien encore les polyphénols, les extraits de raisin, les extraits de pin, les extraits d'olives (comme par exemple le MANOLIVA ^{™}), les extraits de marc, les protéines N-acylées, les hydrolysats totaux de protéines N-acylés, les acides aminés, les polyols tels que la glycérine ou le butylène glycol, l'urée, l'acide pyrrolidonecarboxylique ou un dérivé de cet acide, l'acide glycyrrhétinique, l'alpha-bisabolol, les sucres ou les dérivés des sucres, les polysaccharides ou leurs dérivés, les hydroxyacides, les vitamines, les dérivés de vitamines (comme par exemple le SEPIVITAL^{™}), les enzymes, les co-enzymes (comme par exemple le Coenzyme Q10 ^{™}), les hormones ou "hormone like" (comme par exemple le Phytoage ^{™}), les extraits végétaux tels que les extraits de melon d'eau, les extraits de trèfle d'eau, extraits riches en tanins, les extraits de menthe aquatique, les extraits d'algues d'eau douce ou marine, les cires essentielles, les extraits bactériens, les minéraux comme par exemple l'aspartate mixte de potassium et de magnesium, les lipides tels que les céramides ou les phospholipides, l'hydroquinone, l'arbutine, l'acide kojique, les actifs ayant une activité anti-microbienne tels que le Lipacide ^{™} C8G, le Lipacide ^{™} UG, l'Octopirox ^{™}, le Sensiva ^{™} SC50, le Sepicontrol ^{™} A5), les actifs apaisants décrits dans le brevet US6296859, les actifs ayant une propriété énergisante ou stimulante (par exemple le PHYSIOGENYL ^{™} ou le SEPITONIC ^{™} M3), le panthénol et ses dérivés (comme le SEPICAP ^{™} MP), les minéraux (gamme des GIVOBIO^{™} ou encore le SEPITONIC ^{™} M3).

L'invention sera illustrée par la lecture des exemples non limitatifs suivants.

### EXEMPLE 1 : Procédé de préparation du xylityl-glucoside

On introduit 703,0 g de xylitol dans un réacteur en verre à double enveloppe, dans laquelle circule un fluide caloporteur, et muni d'une agitation efficace.

Le xylitol est fondu à une température de 135°C, et la pâte visqueuse ainsi obtenue est refroidie à 115°C.

Le glucose est alors ajouté progressivement au milieu réactionnel pour permettre sa dispersion homogène.

On ajoute au mélange ainsi obtenu un système catalytique acide constitué de 1,29 g d'acide sulfurique à 96 %.

Le milieu réactionnel est placé sous un vide partiel de 90 mbars à 45 mbars, et maintenu à une température de 100°C-105°C pendant une durée de 4 h 30 avec évacuation de l'eau formée au moyen d'un montage de distillation.

Le milieu réactionnel est ensuite refroidi à 95°C-100°C et neutralisé par ajout de 5 g de soude à 30 %, pour amener le pH d'une solution à 1 % de ce mélange à une valeur de 5,0.

Les caractéristiques du mélange ainsi obtenu sont les suivantes :
Aspect (visuel) : cire orange à température ambiante ;
pH solution à 1 % : 5,0 ;
xylitol résiduel : 55,8 % ;
glucose résiduel : < 1 %.

### EXEMPLE COMPARATIF : Procédé de préparation du glycéryl-glucoside

On introduit 1650,0 g de glycérol dans un réacteur en verre à double enveloppe, dans laquelle circule un fluide caloporteur, et muni d'une agitation efficace.

Le glycérol est porté à 80°C et 646,0 g de glucose anhydre sont dispersés progressivement jusqu'à l'obtention d'un milieu fluide et homogène.

Le milieu réactionnel est maintenu sous agitation pendant une durée de 30 minutes à 85°C, puis 4,65 g d'acide sulfurique à 98% sont introduits.

Le milieu réactionnel est alors amené à 100°C, mis sous vide partiel entre 60 et 30 mbars, et maintenu 4h00 avec évacuation concomitante de l'eau formée *in situ* par la réaction.

Le milieu réactionnel est alors refroidi à environ 80°C et neutralisé par un ajout de 24 g d'une solution d'hydroxyde de soude à 30% pour amener le pH d'une solution à 1% de ce mélange à une valeur de 6,1.

La composition ainsi obtenue présente les caractéristiques suivantes :
- aspect (visuel) : liquide jaune visqueux
- pH solution à 1% : 6,1
- glycérol résiduel : 42,4%
- glucose résiduel : < 1%

### MISE EN EVIDENCE DES PROPRIETES DU POLYOLS-GLYCOSIDE UTILE SELON L'INVENTION

Les propriétés hydratantes du polyols-glycoside utile dans le cadre de l'invention ont été mises en évidence :
- d'une part, par mesure in vivo de l'hydratation cutanée chez le volontaire sain au moyen d'un appareil connu sous la dénomination Hydrascan^{®} ; et
- d'autre part, par mesure in vitro de l'effet du polyols-glycoside sur la production d'acide hyaluronique, composé de la famille des glucosamino glycanes capable de fixer jusqu'à mille fois son poids en eau.

### A - Mesure in vivo de_ l'hydratation cutanée chez le volontaire sain par Hydrascan^{®}

On a mesuré et comparé chez l'homme l'effet sur le taux d'hydratation cutanée du polyols-glycoside selon l'invention, du glycéryl-glucoside et de différents polyols.

### a) Principe de la méthode

Le taux d'hydratation cutanée est mesuré à l'aide de l'appareil commercialisé sous la dénomination Hydrascan^{®}.

Cet appareil, bien connu de l'homme du métier, permet de mesurer le transfert thermique transitoire, paramètre proche de l'effusivité thermique, propriété que possède un corps d'échanger de la chaleur avec un autre corps avec lequel il est mis en contact.

Cet appareil comporte un micro-effusimètre relié à un capteur souple et permet de produire une onde thermique qui se propage dans l'épiderme et d'enregistrer la variation de température pendant l'impulsion.

Le réglage de l'appareil permet de mesurer le taux d'hydratation à trois niveaux de profondeur dans l'épiderme :
Cycle 1 : stratum corneum et épiderme superficiel ;
Cycle 2 : épiderme superficiel et épiderme moyen ;
Cycle 3 : ensemble de l'épiderme.

Les mesures réalisées à l'aide de cet appareil permettent donc d'explorer les couches superficielles de la peau et de mesurer ainsi le taux d'hydratation cutanée de l'ensemble de l'épiderme.

### b) Produits testés

Les produits testés ont été formulés sous forme de gel crème contenant :
- 3 % (poids/volume) du produit à tester ;
- 2 % (poids/volume) de SEPIGEL^{®} 305 (polyacrylamide/ isoparaffine C13-C14/laureth-7) ;
- 5 % (poids/volume) de LANOL^{®} 99 (isononyl isononanoate) ;
- 0,5 % (poids/volume) de SEPICIDE^{®} HB (Phenoxyethanol, Methyl-, Ethyl-, Propyl-, Butylparaben).

Un gel crème de même composition, mais ne contenant aucun produit à tester est utilisé à titre de placebo.

On a ainsi mesuré et comparé le taux d'hydratation cutanée des produits suivants :
Glucose, xylose, glycérol, xylitol, érythritol, produit de l'Exemple 1, produit de l'Exemple comparatif.

### c) Protocole expérimental

L'étude est réalisée sur trois groupes de six volontaires en double aveugle, c'est-à-dire que ni l'expérimentateur, ni le volontaire ne connaissent l'identité du produit à tester.

On définit sur les avant-bras de chaque volontaire les quatre zones cutanées suivantes :
- une zone traitée par un polyol-glucoside ;
- une zone traitée par le polyol correspondant ;
- une zone traitée par le placebo commun à tous les produits ;
- une zone non traitée.

Les produits sont appliqués de façon topique à raison de 20 mg/cm², les mesures étant effectuées 8 h après application.

Pour éviter des variations indésirables dans les mesures, les volontaires sont placés pendant au moins 30 min dans une pièce contrôlée en température (25°C +/- 2°C) et en hygrométrie (50 % +/- 4 %).

Le taux d'hydratation est mesuré à l'aide de l'Hydrascan^{®}, sur chaque zone cutanée définie précédemment ; les valeurs obtenues pour chacun des trois cycles étant enregistrées et exprimées en pourcentage d'augmentation de l'hydratation cutanée par rapport à la zone traitée avec le placebo. Ces valeurs correspondent à la moyenne obtenue pour les six volontaires.

### d) Résultats obtenus

| | **Taux d'hydratation cutanée mesuré à l'Hydrascan^{®}** | | |
|---|---|---|---|
| **Produit** | **Cycle 1 :** stratum comeum + épiderme superficiel | **Cycle 2 :** épiderme superficiel + moyen | **Cycle 3 :** ensemble de l'épiderme |
| Glucose | <5% | <5% | <5% |
| Xylose | <5% | <5% | <5% |
| Glycérol (3 OH) | +14% | +13% | +13% |
| Erythritol (4 OH) | +9% | +9% | +9% |
| Xylitol (5 OH) | <5% | <5% | <5% |
| Exemple comparatif | <5% | <5% | <5% |
| Exemple 1 | +28% | +33% | +30% |

### e) Analyse des résultats-Conclusions

Les résultats, reportés dans le tableau précédent, montrent que le glycérol augmente le taux d'hydratation des couches superficielles et moyennes de l'épiderme ainsi que de l'ensemble de l'épiderme. L'augmentation est comparable quelle que soit la couche épidermique étudiée.

En revanche, cette augmentation est très sensiblement diminuée lorsque le glycérol est éthérifié par le glucose (produit de l'exemple comparatif).

Le glucose et le xylose seuls ne présentent aucune efficacité hydratante.

Le xylitol (polyol présentant cinq groupes hydroxyles) n'a pas d'effet sur le taux d'hydratation des différentes couches de l'épiderme. En revanche, le xylityl glucoside (produit de l'Exemple 1) augmente de façon très importante le taux d'hydratation des couches superficielle et moyenne de l'épiderme ainsi que de l'ensemble de l'épiderme. L'augmentation est supérieure, de l'ordre de 14 à 20 %, à celle obtenue avec le glycérol.

L'érythritol (polyol présentant quatre groupes hydroxyles) augmente le taux d'hydratation des couches superficielle et moyenne de l'épiderme ainsi que de l'ensemble de l'épiderme. Mais l'effet est moins important que celui obtenu avec le glycérol.

En conclusion, cette étude :
- montre le pouvoir hydratant du glycérol et de l'érythritol et confirme que ce pouvoir hydratant diminue lorsque le nombre de groupes hydroxyles du polyol augmente ;
- met en évidence le très fort potentiel hydratant du xylityl glucoside et, à un niveau légèrement moins important, celui de l'érythrityl glucoside. Ces deux potentiels hydratant sont supérieurs à celui du glycérol et du glycéryl glucoside.

### B - Mesure in vitro de l'effet des polyols-glycosides selon l'invention sur la production d'acide hyaluronique

Pour confirmer l'activité hydratante du polyols glycoside selon l'invention, on a mesuré l'effet de ces produits sur le taux d'acide hyaluronique. On sait en effet que l'acide hyaluronique est un glycoaminoglycane non sulfaté majoritaire qui joue un rôle essentiel dans l'hydratation de la peau, par sa capacité à fixer jusqu'à 1 000 fois son poids en eau.

### a) Principe de la méthode

Le taux d'acide hyaluronique est mesuré dans des cultures de fibroblastes dermiques humains normaux.

Les cellules sont incubées, pendant 5 jours, en présence des produits à tester solubilisés dans le milieu d'incubation.

A l'issue de cette incubation, les milieux extracellulaires, dans lesquels l'acide hyaluronique est sécrété, sont prélevés.

L'acide hyaluronique est coloré à l'aide d'un colorant spécifique, le STAINS ALL ((1-éthyl-2-[3-(1-éthylnaphtho-[1,2-d]thiazolin-2-ylidène)-2-méthylpropényl]naphtho-[1,2-d]thiazolium, bromide, fourni par SIGMA), qui interagit avec ce dernier pour produire un changement de spectre d'absorption entre 620 et 660 nm, observé par spectrophotométrie. Une gamme d'étalonnage de l'acide hyaluronique est réalisée en parallèle.

### b) Produits testés

Les polyols, les polyols-glucosides sont testés, en solution aqueuse, à 0,01% et 0,1% (p/v).

Les polyols testés sont le glycérol, le xylitol et l'érythritol. Les polyols-glucosides testés sont le glycéryl-glucoside (produit de l'Exemple comparatif), et le xylityl-glucoside (produit de l'Exemple 1).

### c) Protocole expérimental :

Celui-ci peut être résumé par le schéma suivant : 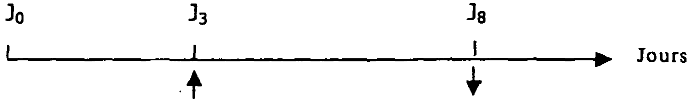 dans lequel J0, J3 et J8 ont les significations suivantes :
J0 : ensemencement des fibroblastes (plaques de culture de 24 puits, 15 300 cellules/puits)
J3 : incubation des produits testés, dilués dans le milieu d'incubation des fibroblastes
J8 : prélèvement des milieux d'incubation des fibroblastes, dosage de l'acide hyaluronique

A l'issue des 5 jours d'incubation en présence des produits, les milieux d'incubation sont prélevés et incubés en présence du STAINS ALL. La réaction colorimétrique est révélée par addition d'eau.

La quantification se fait par spectrophotométrie pour une longueur d'onde de 630 nm.

Une gamme d'étalonnage de l'acide hyaluronique (0 à 12,5 µg/ml) est réalisée en parallèle.

Les résultats sont exprimés en µg/ml d'acide hyaluronique extracellulaire.

### d) Résultats obtenus :

Les résultats qui ont été obtenus, exprimés en pourcentage d'augmentation de la quantité d'acide hyaluronique extracellulaire par rapport au groupe témoin, sont reportés au tableau suivant :

| | **Concentration (%, p/v)** | |
|---|---|---|
| **Produit** | **0,01** | **0,1** |
| Glucose | < 10% | <10% |
| Glycérol | < 10% | < 10% |
| Erythritol | < 10% | < 10% |
| Xylitol | <10% | < 10% |
| Produit de l'exemple comparatif | <10% | < 10% |
| Produit de l'exemple 1 | +158% | +161% |

### e) Analyse des résultats - Conclusions

Après 5 jours d'incubation en présence des fibroblastes, aucun des polyols testés n'augmente le taux d'acide hyaluronique extracellulaire.

Le glycéryl-glucoside (produit de l'Exemple comparatif) n'augmente pas le taux d'acide hyaluronique extracellulaire.

Le xylityl-glucoside (produit de l'exemple 1), augmente le taux d'acide hyaluronique extracellulaire.

Parmi les polyols-glucosides testés, le xylityl-glucoside (produit de l'exemple 1) est le plus efficace.

Les trois polyols testés, glycérol, xylitol et érythritol n'ont pas d'effet sur le taux extracellulaire en acide hyaluronique.

Parmi les polyol-glucosides testés, glycéryl-glucoside, et xylityl-glucoside, le xylityl-glucoside augmente de façon très marquée le taux extracellulaire en acide hyaluronique. Ce modèle in vitro permet de sélectionner le xylityl-glucoside comme étant le produit le plus intéressant ; ce classement est similaire à celui obtenu dans le test in vivo.

Les propriétés restructurantes du polyols-glycoside utile dans le cadre de l'invention ont été mises en évidence par mesure in vitro de l'effet des polyols-glycosides, en particulier le xylityl-glucoside, sur la synthèse du céramide 1 et du céramide 2, composés de la famille des lipides épidermiques qui jouent un rôle clé dans la fonction barrière de la peau.

### C- Mesure in vitro de l'effet du xylityl-glucoside, selon l'invention, sur la synthèse des céramides épidermiques

Pour illustrer l'augmentation de la cohésion cellulaire de la peau par les polyols-glycosides, en particulier par le xylityl-glucoside selon l'invention, on a mesuré l'effet de ces produits sur la synthèse du céramide 1 et du céramide 2 en comparaison avec le glycérol et des composés connus par l'homme de l'art pour augmenter cette synthèse.

### a) Principe de la méthode

L'étude est réalisée in vitro dans un modèle d'explant de peau humaine. Les produits, formulés à 3% dans un gel crème, sont appliqués à la surface des explants de peau pendant 24 heures. La néosynthèse des lipides épidermiques est étudiée par un marquage radioactif (acétate marqué au carbone 14) des lipides néosynthétisés suivi d'une chromatographie en couche mince pour séparer les différents types de lipides et en particulier les céramides.

### b) Produits testés

- Le xylityl-glucoside selon l'exemple 1 de l'invention, formulé à 3% dans un gel crème comprenant 2% de Sepigel^{®} 305, 5% de Lanol 99, qsp. d'eau.
- Le glycérol formulé à 3% dans le même gel crème.
- Le facteur de croissance épidermique (EGF), connu pour augmenter la synthèse des céramides, qui constitue une molécule de référence pour ce test. L'EGF est testé à 10 ng/ml dans le milieu de culture des explants de peau.
- Un placebo correspondant au gel crème.
- Une formulation commercialisée contenant de l'acide lactique, qui constitue une référence pour le test ; l'acide lactique étant connu pour augmenter la synthèse des céramides.

### c) Protocole expérimental

L'étude est réalisée sur des disques de peau humaine provenant de chirurgie esthétique (plastie abdominale, femme caucasienne âgée de 35 ans). Des disques de 8 mm de diamètre sont réalisés à l'emporte-pièce et déposés sur des nacelles de culture disposées dans des puits de culture contenant un milieu nutritif approprié (milieu MEM/M199 (¾, ¼, v/v) additionné de pénicilline (50 UI/ml), de streptomycine (50 µg/ml), de bicarbonate de sodium (0,2% p/v), de sérum (2%, v/v) et d'acétate marqué au carbone 14 (1 µCi/ml)).

Les produits sont testés en application topique (sauf pour l'EGF) pendant 24 heures.

A l'issue des 24 heures d'incubation, les explants de peau sont rincés avec du tampon phosphate salin. Pour chaque disque de peau, le derme est dissocié de l'épiderme par un choc thermique contrôlé (1 min à 70°C). Les lipides épidermiques sont extraits par partition entre une phase organique (méthanol/chloroforme (1:2)) et une phase aqueuse (chlorure de potassium à 0,25 M). La phase organique est ensuite évaporée sous vide et le résidu est repris dans un mélange chloroforme/méthanol (2 :1).

Les différents lipides épidermiques sont alors séparés par une chromatographie en couche mince (silice 60) : chloroforme/ acétone/méthanol (38 :2 :10) ; chloroforme/acétone/méthanol (40 :5 :5) ; chloroforme/acétate d'éthyle/éther diéthylique/ méthanol (36 :10 :3 :1). La radioactivité des fractions ainsi séparées est comptée avec un analyseur de radioactivité (STORM, AMERSHAM).

Les résultats sont exprimés en % de variation par rapport au groupe témoin.

### d) Résultats obtenus

Les résultats qui ont été obtenus, exprimés en pourcentage d'augmentation de la quantité de céramide 1 et de céramide 2 par rapport au groupe témoin, sont reportés dans le tableau suivant :

| | EGF | Formulation à l'adde lactique | Xylityl-glucoside selon l'exemple 1 | Glycérol | Placebo |
|---|---|---|---|---|---|
| Céramide 1 | 166,1% | 158,5% | 295,7% | 114,9% | 174,6% |
| Céramide 2 | 152,3% | 125,6% | 236,5% | 151,7% | 169,9% |

### e) Analyse des résultats-Conclusion

- L'utilisation du xylityl-glucoside dans le gel crème augmente de façon significative la néosynthése des céramides 1 et 2, laquelle n'est pas observée en présence du glycérol et du placebo dans le même schéma de formulation.
- L'EGF et la formulation contenant de l'acide lactique, connus par l'homme de l'art comme possédant une action sur l'augmentation de la néosynthèse des céramides 1 et 2, agissent, mais de façon moins efficace que la composition issue de l'exemple 1 de invention.

Ces effets sur les céramides sont significatifs d'un effet restructurant du xylityl-glucoside sur la barrière cutanée, ce qui est d'ailleurs en accord avec les mesures d'hydratation réalisées in vivo dans les différentes couches de l'épiderme. Ces résultats sont en adéquation avec les effets hydratants à long terme du xylityl-glucoside.

### MISE EN EVIDENCE DE LA TOLERANCE CUTANEE DU POLYOLS-GLYCOSIDE UTILE SELON L'INVENTION

La tolérance cutanée des différents polyols-glycosides a été évaluée par une étude d' « évaluation cutanée de l'irritation cutanée aiguë», réalisée par une société indépendante de conseils-expertises pharmaceutiques et cosmétiques.

Les mesures d'Indices d'Irritation Primaire Cutanée (Ipc), menées selon le même protocole, sont contenues dans le tableau suivant :

| **Produit** | **IRRITATION** | |
|---|---|---|
| | Indice Ipc | Classement |
| Xylityl-Glucoside (exemple 1) | 0 | Non irritant |
| Glycéryl-Glucoside (exemple comparatif) | 0 | Non irritant |

Chaque composé étudié, au vu des résultats obtenus dans les conditions expérimentales retenues, sont classés non irritant pour la peau, en référence au barème proposé dans le protocole décrit *au* Journal Officiel de la République Français du 21 Février 1981*.*

Ces nouvelles compositions à base du polyols-glycosides (exemple 1) n induisent donc pas de modification de la tolérance cutanée par rapport à l'exemple comparatif relatif un état de l'art antérieur.

On donnera maintenant quelques exemples de compositions à activité hydratante selon l'invention.

### EXEMPLE 3 : LAIT CORPOREL HYDRATANT

### Formule

| | | |
|---|---|---|
| **A** | Eau | QSP |
| | | 100% |
| | FUCOGEL | 03.00 % |
| | MICROPEARL™ M305 (Methylmethacrylate crosspolymer) | 05.00 % |
| | Actif Hydratant | 03.00 % |
| | MONTANOV™ L (C14-22 Alcohol & C12-20 Alkyl glucoside) | 04.00 % |
| | | |
| **B** | LANOL™ 99 (Isononyl isononanoate) | 04.00 % |
| | SIMULGEL™ EG (sodium acrylate sodium acryloyldimethyl taurate copolymer/ Isohexadécane/ Sorbitan oleate) | 01.00 % |
| | | |
| **C** | DC345 (cyclométhicone) | 12.00 % |
| **D** | Parfum | Qs. |
| | SEPICIDE™ HB | 00.30 % |
| | (Phenoxyethanol/Methylparaben/Ethylparaben/Propylpara ben Butylparaben) | |
| | SEPICIDE™ CI (Imidazolidinyl urea) | 00.20 % |

### Mode opératoire :

- chauffer les phases grasses et aqueuses (B et A) de façon séparée à 75°C-80°C
- émulsionner B dans A sous agitation avec une turbine rotor-stator
- ajouter C et maintenir sous forte agitation pendant quelques minutes
- refroidir sous agitation modérée
- ajouter D à 30°C

### EXEMPLE 4 : GEL-CREME HYDRATANT

### Formule

| | | |
|---|---|---|
| **A** | Eau | QSP 100% |
| | Glycérine | 02.50 % |
| | MICROPEARL™ M305 | 01.00 % |
| | SEPICIDE™ CI | 00.20% |
| | Actif Hydratant | 02.00 % |
| | | |
| **B** | SIMULGEL™ EG | 01.00 % |
| | | |
| **C** | LANOL™ 99 | 05.00 % |
| | DC345 | 02.50 % |
| | SEPICIDE™ HB | 00.30 % |
| | Parfum | Qs. |

### Mode opératoire :

- disperser à température ambiante le Micropear™ M305 dans le mélange eau, glycérine, actif hydratant, Sepicide™ CI
- ajouter la phase A préparée selon la méthode évoquée ci-dessus, sur B de façon progressive en homogénéisant la préparation après chaque ajout sous agitation modérée
- ajouter C dans le gel précédemment préparé.

**Caractéristiques :** Aspect : gel blanc brillant ; pH = 6,2 ;

### EXEMPLE 5 : EAU CORPORELLE ENERGISANTE HYDRATANTE

### Formule :

| | | |
|---|---|---|
| **A** | Parfum | Qs. |
| | ORAMIX™ CG 110 | 02.50% |
| | SEPICIDE™ HB | 00.50 % |
| | | |
| **B** | Glycérine | 01.00 % |
| | Actif Hydratant | 1,00% |
| | SEPITONIC™ M3 | 01.00 % |
| | SEPICIDE™ CI | 00.30 % |
| | Eau | QSP 100 % |

### Mode opératoire :

- solubiliser le parfum et le SEPICIDE™ HB dans l'ORAMIX™ CG 110 pour préparer A
- ajouter les ingrédients de B dans l'ordre indiqué à température ambiante sous agitation modérée.

**Caractéristiques :** Aspect : liquide limpide translucide et incolore ; pH = 5.

### EXEMPLE 6 : GEL DOUCHE ENERGISANT

### Formule

| | | |
|---|---|---|
| **A** | Actif hydratant | 03.00 % |
| | SEPICIDE™ HB | 00.30 % |
| | Parfum | Qs. |
| | MONTANOX™ 81 (polysorbate 81) | 02.00 % |
| **B** | PROTEOL™ OAT (sodium lauroyl OAT aminoacids) | 05.00 % |
| | sodium lauryl éther sulfate 28 % | 45.00% |
| | SEPITONIC™ M3 | 01.00 % |
| | SEPICIDE™ CI | 00.30 % |
| | Eau | QSP 100 % |
| | MONTALINE™ C40 (Cocamidopropylalbetainamide MEA chloride) | 05.00 % |
| | chlorure de sodium | 00.75 % |
| | acide lactique | Qs. pH |

### Mode opératoire :

- mélanger les ingrédients de la phase A à température ambiante, sous agitation modérée
- ajouter les ingrédients de la phase B dans l'ordre indiqué dans les mêmes conditions opératoires.

**Caractéristiques :** Aspect : gel limpide ; pH = 6,5.

### EXEMPLE 7 : ROUGE A LEVRE HYDRATANT

### Formule :

| | | |
|---|---|---|
| **A** | Huile de ricin | Qs |
| | | 100.00% |
| | *Cera alba* (bee wax) | 07.50% |
| | Cire de Candelilla | 07.50% |
| | *Cera microcristallina* | 15.00% |
| | SEPIFEEL™ ONE (Palmitoylproline/Magnesium palmitoylglutamate/ sodiumpalmitoylsarcosinate-SEPPIC) | 03.00% |
| | SEPILIFT™ DPHP (DiPalmitoylHydroxyProline- SEPPIC) | 01.00% |
| | Cetyl Alcohol | 01.50% |
| | Isopropyl Lanolate | 01.00% |
| | Cetyl Ricinoleate | 00.80% |
| | Micropearl™ M 310 (crosslinked PMMA, distribué par SEPPIC) | 02.00% |
| | *Butyrospermum Parkii* (Beurre de Karité) | 03.00% |
| | Paraffinum liquidum | 02.50% |
| | LANOL™ 1688 (cetearyl octanoate- SEPPIC) | 02.50% |
| | Caprylic/Capric Triglyceride | 04.00% |
| | Cire de Carnauba | 03.50% |
| | CI 77491 | 01.40% |
| | CI 45410-DC red 27 | 00.10% |
| | CI 77891-Titanium dioxide | 11.00% |
| | Perfluoromethyl isopropyl ether | 00.10% |
| | | |
| **B** | MONTANE™ 80 | 47.50% |
| | Eau | Qs |
| | | 100.00% |
| | Actif hydratant | 05.00 % |
| | SEPICIDE™ CI | 00.20% |
| | SEPICIDE™ HB | 00.30 % |

### Mode opératoire :

- la phase A est préparée dans un broyeur tricylindrique, en ajoutant chaque composé préalablement préparé sous forme fondue.
- la phase B est ajoutée à 80°C sous agitation modérée jusqu'à l'obtention d'une dispersion homogène sur la phase fondue
- le mélange est ensuite coulé dans des moules adaptés pour la mise en forme.

## Revendications

1. Compositions à usage topique, **caractérisées en ce qu'**elles contiennent une quantité efficace d'un polyol-glycoside obtenu par acétalisation du xylitol de formule : 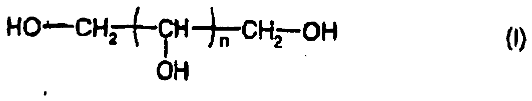 dans laquelle n est un nombre entier égal à 3, avec le glucose.

2. Compositions selon la revendication 1 **caractérisées en ce qu'**il s'agit d'une composition cosmétique, une composition pharmaceutique, une composition dermopharmaceutique ou une composition d'imprégnation pour lingette.

3. Compositions selon la revendication 1 ou 2, **caractérisées en ce qu'**elle se présentent sous forme de solution, d'émulsion ou de micro-émulsion du type eau-dans-huile (E/H) ou huile-dans-eau (H/E), d'émulsion multiple de type eau-dans-huile-dans-eau (E/H/E) ou huile-dans-eau-dans-huile (H/E/H), de gel, d'hydro-dispersion, de bâton solide, de pommade, d'aérosol ou encore sous forme anhydre comme une poudre.

4. Utilisation en tant qu'agent d'hydratation des couches supérieures de l'épiderme, d'un polyol-glycoside obtenu par acétalisation du xylitol de formule 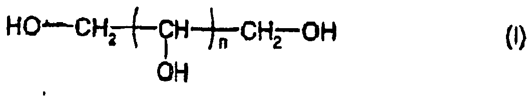 dans laquelle n est un nombre entier égal à 3, avec le glucose.

5. Utilisation en tant qu'agent restructurant de l'épiderme, d'un polyol-glycoside obtenu par acétalisation du xylitol de formule 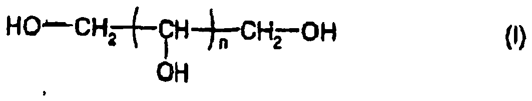 dans laquelle n est un nombre entier égal à 3, avec le glucose. ».

## Patentansprüche

1. Zusammensetzungen zur topischen Verwendung, **dadurch gekennzeichnet, dass** sie eine wirksame Menge eines Polyol-Glykosids enthalten, welches erhalten wurde, indem Xylit der folgenden Formel: 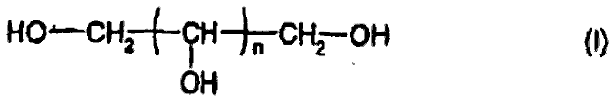 wobei n eine ganze Zahl gleich 3 ist, mit Glucose acetalisiert wurde.

2. Zusammensetzungen nach Anspruch 1, **dadurch gekennzeichnet, dass** es sich um eine kosmetische Zusammensetzung, eine pharmazeutische Zusammensetzung, eine dermopharmazeutische Zusammensetzung oder eine Zusammensetzung zur Tränkung von Wischtüchern handelt.

3. Zusammensetzungen nach Anspruch 1 oder 2, **dadurch gekennzeichnet, dass** sie in Form einer Lösung, einer Emulsion oder einer Mikroemulsion des Typs Wasser-in-Öl (W/O) oder Öl-in-Wasser (O/W), einer multiplen Emulsion des Typs Wasser-in-Öl-in-Wasser (W/O/W) oder Öl-in-Wasser-in-Öl (O/W/O), eines Gels, einer Hydrodispersion, eines feststofflichen Stifts, einer Salbe, eines Aerosols oder auch wasserfrei, wie etwa in Form eines Pulvers, vorliegen.

4. Verwendung eines Polyol-Glykosids, welches erhalten wurde, indem Xylit der folgenden Formel 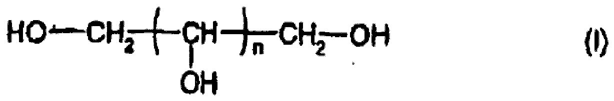 wobei n eine ganze Zahl gleich 3 ist, mit Glucose acetalisiert wurde, als Mittel zur Hydratation der oberen Schichten der Epidermis.

5. Verwendung eines Polyol-Glykosids, welches erhalten wurde, indem Xylit der folgenden Formel 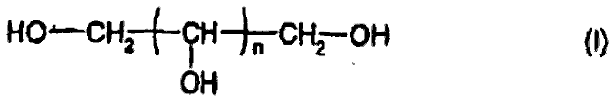 wobei n eine ganze Zahl gleich 3 ist, mit Glucose acetalisiert wurde, als Aufbaumittel für die Epidermis.

## Claims

1. Compositions for topical use, **characterised in that** they contain an effective quantity of a polyol-glycoside obtained by acetalisation of the xylitol of formula: 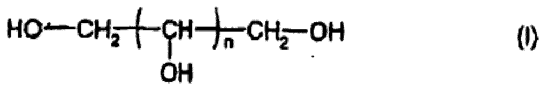 wherein n is an integer equal to 3, with glucose.

2. Compositions according to claim 1, **characterised in that** the composition is a cosmetic composition, a pharmaceutical composition, a dermopharmaceutical composition or an impregnating composition for wipes.

3. Compositions according to claim 1 or 2, **characterised in that** they take the form of a solution, emulsion or micro-emulsion of the water-in-oil (W/O) type or of the oil-in-water (O/W) type, a multiple emulsion of the water-in-oil-in-water (W/O/W) or oil-in-water-in-oil (O/W/O) type, a gel, an aqueous dispersion, a solid bar, an ointment, an aerosol or an anhydrous form such as a powder.

4. Use of a polyol-glycoside obtained by acetalisation of the xylitol of formula: 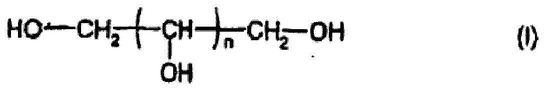 wherein n is an integer equal to 3, with glucose, as a moisturiser for the upper layers of the epidermis.

5. Use of a polyol-glycoside obtained by acetalisation of the xylitol of formula: 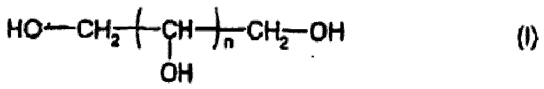 wherein n is an integer equal to 3, with glucose, as a restructuring agent for the epidermis.
